# EUROPEAN PATENT APPLICATION

(11) **EP 0 999 267 A1**
(43) Date of publication of application: **10.05.2000**
(21) Application number: 99120934.7
(22) Date of filing: 02.11.1999
(51) Int. Cl.: C12N 1/20, C12P 13/10, C12N 15/52

(54) **Method for producing L-arginine**

(30) Priority: 02.11.1998 JP 31230198; 24.09.1999 JP 27120499
(71) Applicant: Ajinomoto Co., Inc., Tokyo 104 (JP)
(72) Inventor: Suga, Mikiko, Kawasaki-shi, Kanagawa (JP); Kuwabara, Yoko, Kawasaki-shi, Kanagawa (JP); Hashiguchi, Kenichi, Kawasaki-shi, Kanagawa (JP); Ito, Hisao, Kawasaki-shi, Kanagawa (JP); Nakamatsu, Tsuyoshi, Kawasaki-shi, Kanagawa (JP); Kurahashi, Osamu, Kawasaki-shi, Kanagawa (JP)
(74) Representative: HOFFMANN - EITLE

(57) **Abstract**

Disclosed is a coryneform bacterium having L-arginine-producing ability in which an activity of intracellular arqininosuccinate synthase is enhanced, wherein the activity of intracellular argininosuccinate synthase is enhanced by, for example, increasing copy number of a gene which codes for an argininosuccinate synthase derived form a coryneform bacterium in the bacterial cell, or modifying an expression regulation sequence for the gene in the bacterial cell so that expression of the gene should be enhanced. L-Arginine is produced by culturing the bacterium having L-arginine-producing ability in a medium so that L-arginine should be produced and accumulated, and collecting the L-arginine from the medium. The present invention provides a coryneform bacterium of improved L-arginine-producing ability and an efficient method for producing L-arginine.

### Annex to the description

SEQUENCE LISTING

<110> Ajinomoto Co., Ltd

<120> Method for Producing L-Arginine

<130> OP913

<141> 1999-  -

<150> JP 10-312301
<151> 1998-11-02

<150> JP 11-271204
<151> 1999-09-24

<160> 15

<170> PatentIn Ver. 2.0

<210> 1
<211> 30
<212> DNA
<213> Artificial Sequence

<220>
<223> Description of Artificial Sequence:primer

<400> 1
agaccgccgg agtatgcaag aacgatgcgg                                        30

<210> 2
<211> 30
<212> DNA
<213> Artificial Sequence

<220>
<223> Description of Artificial Sequence:primer

<400> 2
gacttcacca tcaatcatct tcttcaggta                                        30

<210> 3
<211> 30
<212> DNA
<213> Artificial Sequence

<220>
<223> Description of Artificial Sequence:primer

<400> 3
accttcgacc agaccctggc taagggcttt                                        30

<210> 4
<211> 30
<212> DNA
<213> Artificial Sequence

<220>
<223> Description of Artificial Sequence:primer

<400> 4
gctaacaagc gcgatcgcga agctggcaac                                        30

<210> 5
<211> 30
<212> DNA
<213> Artificial Sequence

<220>
<223> Description of Artificial Sequence:primer

<400> 5
gcgatgacac cgtttttgtt ctcgc                                             25

<210> 6
<211> 30
<212> DNA
<213> Artificial Sequence

<220>
<223> Description of Artificial Sequence:primer

<400> 6
ggcgacatcc ttgcccagat gatca 25

<210> 7
<211> 25
<212> DNA
<213> Artificial Sequence

<220>
<223> Description of Artificial Sequence:primer

<400> 7
gacttcacca tcaatcatct tcttc 25

<210> 8
<211> 23
<212> DNA
<213> Artificial Sequence

<220>
<223> Description of Artificial Sequence:primer

<400> 8
gccaggtaca actgtctgaa ttg 23

<210> 9
<211> 40
<212> DNA
<213> Artificial Sequence

<220>
<223> Description of Artificial Sequence:primer

<400> 9
gttaatcgct tgccaatgca ggcaggtaag gtataacccg 40

<210> 10
<211> 40

<212> DNA
<213> Artificial Sequence

<220>
<223> Description of Artificial Sequence:primer

<400> 10
gttaatcgct tgctaatgca ggcaggtaag gtataacccg 40

<210> 11
<211> 40
<212> DNA
<213> Artificial Sequence

<220>
<223> Description of Artificial Sequence:primer

<400> 11
gttaatcgct tgtcaatgca ggcaggtaag gtataacccg 40

<210> 12
<211> 40
<212> DNA
<213> Artificial Sequence

<220>
<223> Description of Artificial Sequence:primer

<400> 12
gttaatcgct tgttaatgca ggcaggtaag gtataatccg 40

<210> 13
<211> 40
<212> DNA
<213> Artificial Sequence

<220>
<223> Description of Artificial Sequence:primer

<400> 13
gttaatcgct tgtcaatgca ggcaggtaag gtataatccg 40

<210> 14
<211> 30
<212> DNA
<213> Artificial Sequence

<220>
<223> Description of Artificial Sequence:primer

<400> 14
gggttccagc ctcgtgcgga attcgtggag                                        30

<210> 15
<211> 25
<212> DNA
<213> Artificial Sequence

<220>
<223> Description of Artificial Sequence:primer

<400> 15
gcgttaccca gagctggatc ctcgg                                             25

## Description

### Technical Field

The present invention relates to an L-arginine-producing coryneform bacterium and a method for producing L-arginine. L-Arginine is an industrially useful amino acid as ingredients of liver function promoting agents, amino acid transfusions, comprehensive amino acid preparations and the like.

### Background Art

Conventional L-arginine production by fermentation has been performed by utilizing wild-type strains of coryneform bacteria; coryneform bacteria resistant to certain agents including sulfa drugs, 2-thiazolealanine, α-amino-β-hydroxyvaleric acid and the like; coryneform bacteria exhibiting auxotrophy for L-histidine, L-proline, L-threonine, L-isoleucine, L-methionine, or L-tryptophan in addition to the resistance to 2-thiazolealanine (Japanese Patent Laid-open No. 54-44096); coryneform bacteria resistant to ketomalonic acid, fluoromalonic acid, or monofluoroacetic acid (Japanese Patent Laid-open No. 57-18989); coryneform bacteria resistant to argininol (Japanese Patent Laid-open No. 62-24075); coryneform bacteria resistant to X-guanidine (X represents a derivative of fatty acid or aliphatic chain, Japanese Patent Laid-open No. 2-186995) or the like.

On the other hand, there have also been disclosed methods for producing L-arginine utilizing recombinant DNA techniques. That is, there have been disclosed methods for producing L-arginine by utilizing a microorganism belonging to the genus *Corynebacterium* or *Brevibacterium* which is made to harbor a recombinant DNA comprising a vector DNA and a DNA fragment containing genes for acetylornithine deacetylase, N-acetylglutamic acid-γ-semialdehyde dehydrogenase, N-acetyl glutamokinase, and argininosuccinase derived from a microorganism belonging to the genus *Escherichia* (Japanese Patent Publication No. 5-23750), a coryneform bacterium harboring a recombinant DNA comprising a vector DNA and a DNA fragment carrying genetic information concerning the syntheses of N-acetylglutamate kinase, N-acetyl-γ-glutamylphosphate reductase, and argininosuccinase derived from an *Escherichia* bacterium, but not carrying genetic information concerning the synthesis of the acetylornithine deacetylase, or a DNA fragment carrying genetic information concerning the syntheses of N-acetyl-γ-glutamylphosphate reductase, argininosuccinase, and argininosuccinate synthase, but not carrying genetic information concerning the synthesis of acetylornithine deacetylase (Japanese Patent Publication No. 7-55155), and a coryneform bacterium harboring a recombinant DNA comprising a vector DNA and a DNA fragment carrying genetic information concerning the syntheses of N-acetylglutamate kinase, N-acetyl-γ-glutamylphosphate reductase, N-acetylornithine-γ-aminotransferase, ornithine carbamyltransferase, an enzyme having activity for restoring non-auxotrophy for arginine in an arginine-auxotrophic N-acetylglutamate synthase deficient mutant strain of *Escherichia coil,* and an enzyme having activity for restoring non-auxotrophy for arginine in an arginine-auxotrophic acetylornithine deacetylase deficient mutant strain of *Escherichia coli* (Japanese Patent Publication No. 7-28749).

In microorganisms such as *Escherichia coil*, L-arginine is biosynthesized from L-glutamic acid via N-acetylglutamate, N-acetylglutamylphosphate, N-acetylglutamic acid semialdehyde, N-acetylornithine, ornithine, citrulline, and argininosuccinate (Sakanyan, V. *et al*., *Micorobiology,* 142, 99-108 (1996)). These intermediates are produced through reactions catalyzed by N-acetylglutamate synthase, N-acetyl glutamate kinase, N-acetylglutamylphosphate reductase, acetylornithine aminotransferase, N-acetylornithinase, ornithine carbamyltransferase, and argininosuccinate synthase, respectively. Then, L-arginine is finally produced through a reaction catalyzed by argininosuccinase. These enzymes are encoded by *argA, argB, argC*, *argD, argE, argF, argG, and argH* genes, respectively.

In the reaction generating ornithine from N-acetylornithine among the aforementioned reactions, the acetyl group is liberated from N-acetylornithine as acetate. In coryneform bacteria, this reaction is coupled to the reaction generating N-acetylglutamate utilizing glutamate as an acceptor so that the acetyl moiety is recycled (acetyl cycle). This reaction is catalyzed by the ornithine acetyltransferase (argJ gene product, mentioned above).

Further, while feedback inhibition by the final product, L-arginine, acts on the N-acetylglutamate synthase which catalyzes the first reaction in *Escherichia coli,* it is said that that inhibition acts on the N-acetylglutamate kinase catalyzing the next reaction in a bacterium having the acetyl cycle like coryneform bacteria (mentioned above).

As described above, although the L-arginine biosynthetic pathways of *Escherichia coli* and coryneform bacteria are similar to each other, each reaction and regulation thereof are not necessarily the same.

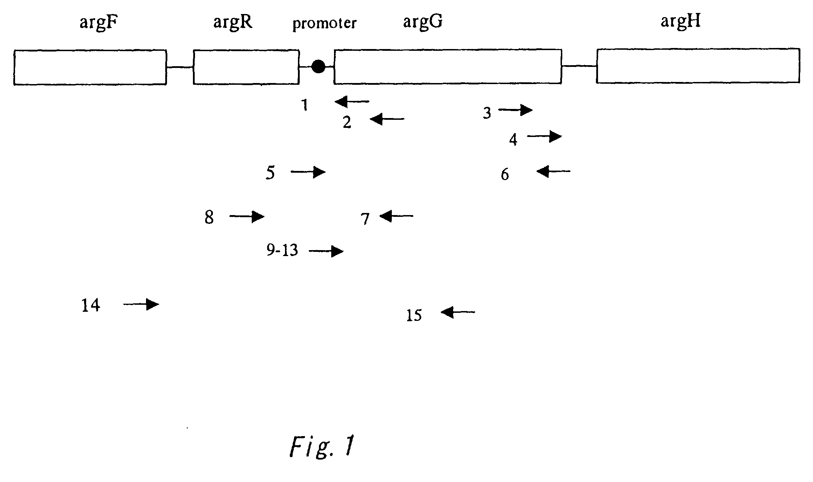

As for ORF of *argG* that encodes the argininosuccinate synthase of coryneform bacteria, there have been reported a nucleotide sequence for the ORF of the gene of *Corynebacterium glutamicum* (GenBank accession AF030520), and a nucleotide sequence for the gene including the flanking regions on the both sides of the ORF (GenBank accession AF049897). While it has also been reported that *argG* of coryneform bacteria suffers repression (see *Agric. Biol. Chem.*, *43*, 1899-1903 (1979), Fig. 1), that report is not based on results of enzymatic activity measurement, and there has not been reported at all that the enzymatic activity itself of the argininosuccinate synthase has been measured in coryneform bacteria. Furthermore, the structure of argininosuccinate synthase gene has been elucidated in *Escherichia coli* and yeast, and in particular, the repressor binding region involved in expression inhibition of *argG* has also been elucidated in detail in Escherichia coli (*Gene*, *95*, 99-104, (1990); *J. Mol. Biol*., *226*, 367-386 (1992)). However, the detail of the expression control of *argG* in coryneform bacteria remains unclear. Moreover, the role of the argininosuccinate synthase in the control of L-arginine biosynthesis in coryneform bacteria is also uncertain.

### Summary of the Invention

An object of the present invention is to improve L-arginine-producing ability of coryneform bacteria, thereby providing an efficient method for producing L-arginine.

In order to achieve the aforementioned object, the present inventors earnestly continued studies. As a result, they found that the L-arginine-producing ability of coryneform bacteria can be improved by introducing a gene coding for the argininosuccinate synthase into the bacteria, which was attempted with giving an eye to the fact that citrulline was accumulated in culture of L-arginine-producing bacteria of the conventional coryneform bacteria, and thus accomplished the present invention.

That is, the present invention provides a coryneform bacterium having L-arginine-producing ability in which an activity of intracellular argininosuccinate synthase is enhanced.

The present invention also provides the aforementioned bacterium, wherein the argininosuccinate synthase is derived from a coryneform bacterium.

The present invention further provides the aforementioned bacterium, wherein the activity of intracellular argininosuccinate synthase is enhanced by increasing copy number of a gene which codes for an argininosuccinate synthase derived from a coryneform bacterium in the bacterial cell, or modifying an expression regulation sequence for the gene in the bacterial cell so that expression of the gene should be enhanced.

The present invention still further provides a method for producing L-arginine comprising the steps of culturing the aforementioned bacterium in a medium to produce and accumulate L-arginine, and collecting the L-arginine from the medium.

The term "L-arginine-producing ability" used herein means an ability of the microorganism of the present invention to accumulate L-arginine in a medium when it is cultured in the medium.

The coryneform bacteria of the present invention can be utilized as L-arginine-producinq bacteria, or starting materials of breeding of L-arginine-producing bacteria. According to the present invention, L-arginine can be efficiently produced by using coryneform bacteria.

### Brief explanation of the Drawings

Fig. 1 is a drawing showing the geometry of the *argG* gene and the sequences flanking thereto with respect to each primer.

### Detailed Description of the Invention

Hereafter, the present invention will be explained in detail.

### <1> Coryneform bacteria of the present invention

The coryneform bacteria of the present invention are coryneform bacteria that have L-arginine-producing ability, in which intracellular argininosuccinate synthase activity is enhanced. The "coryneform bacteria" referred to in the present invention includes bacteria having been hitherto classified into the genus *Brevibacterium* but united into the genus *Corynebacterium* at present (*Int. J. Syst. Bacteriol., 41*, 255 (1981)), and include bacteria belonging to the genus *Brevibacterium* closely relative to the genus *Corynebacterium.* Examples of such coryneform bacteria include the followings.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium liliun (Corynebacterium glutamicum)*
*Corynebacterium melassecola*
*Corynebacterium thermoaminogenes*
*Corynebacterium herculis*
*Brevibacterium divaricatum (Corynebacterium glutamicum)*
*Brevibacterium flavum (Corynebacterium glutamicum) Brevibacterium immariophilum*
*Brevibacterium factofermentum (Corynebacterium glutamicum)*
*Brevibacterium roseum*
*Brevibacterium saccharolyticum*
*Brevibacterium thiogenitalis*
*Brevibacterium album*
*Brevibacterium cerinum*
*Microbacterium ammoniaphilum*

While the coryneform bacteria that have the L-arginine-producing ability are not particularly limited so long as they have the L-arginine-producing ability, they include, for example, wild-type strains of coryneform bacteria; coryneform bacteria resistant to certain agents including sulfa drugs, 2-thiazolealanine, α-amino-β-hydroxyvaleric acid and the like; coryneform bacteria exhibiting auxotrophy for L-histidine, L-proline, L-threonine, L-isoleucine, L-methionine, or L-tryptophan in addition to the resistance to 2-thiazolealanine (Japanese Patent Laid-open No. 54-44096); coryneform bacteria resistant to ketomalonic acid, fluoromalonic acid, or monofluoroacetic acid (Japanese Patent Laid-open No. 57-18989); coryneform bacteria resistant to argininol (Japanese Patent Laid-open No. 62-24075); coryneform bacteria resistant to X-guanidine (X represents a derivative of fatty acid or aliphatic chain, Japanese Patent Laid-open No. 2-186995) and the like.

Specifically, the following strains can be exemplified.
*Brevibacterium flavum* AJ11169 (BP-6892)
*Corynebacterium glutamicum* AJ12092 (FERM BP-6906)
*Brevibacterium flavum* AJ11336 (FERM BP-6893)
*Brevibacterium flavum* AJ11345 (FERM BP-6894)
*Corynebacterium glutamicum* AJ12430 (FERM BP-2228)

The AJ11169 strain and the AJ12092 strain are the 2-thiazolealanine resistant strains mentioned in Japanese Patent Laid-open No. 54-44096, the AJ11336 strain is the strain having argininol resistance and sulfadiazine resistance mentioned in Japanese Patent Publication No. 62-24075, the AJ11345 strain is the strain having argininol resistance, 2-thiazolealanine resistance, sulfaguanidine resistance, and exhibiting histidine auxotrophy mentioned in Japanese Patent Publication No. 62-24075, and the AJ12430 strain is the strain having octylguanidine resistance and 2-thiazolealanine resistance mentioned in Japanese Patent Laid-open No. 2-186995.

AJ11169 was deposited on August 3, 1977 in National Institute of Bioscience and Human Technology of Agency of Industrial Science and Technology of Ministry of International Trade and Industry (zip code: 305-8566, 1-3 Higashi 1-Chome, Tsukuba-shi, Ibaraki-ken, Japan), as deposition number of FERM P-4161, and transferred from the original deposit to international deposit based on Budapest Treaty on September 27, 1999, and has been deposited as deposition number of FERM BP-6892.

AJ12092 was deposited on September 29, 1983 in National Institute of Bioscience and Human Technology of Agency of Industrial Science and Technology of Ministry of International Trade and Industry, as deposition number of FERM P-7273, and transferred from the original deposit to international deposit based on Budapest Treaty on October 1, 1999, and has been deposited as deposition number of FERM BP-6906.

AJ11336 was deposited on April 25, 1979 in National Institute of Bioscience and Human Technology of Agency of Industrial Science and Technology of Ministry of International Trade and Industry, as deposition number of FERM P-4939, and transferred from the original deposit to international deposit based on Budapest Treaty on September 27, 1999, and has been deposited as deposition number of FERM BP-6893.

AJ11345 was deposited on April 25, 1979 in National Institute of Bioscience and Human Technology of Agency of Industrial Science and Technology of Ministry of International Trade and Industry, as deposition number of FERM P-4948, and transferred from the original deposit to international deposit based on Budapest Treaty on September 27, 1999, and has been deposited as deposition number of FERM BP-6894.

AJ12430 was deposited on December 26, 1988 in National Institute of Bioscience and Human Technology of Agency of Industrial Science and Technology of Ministry of International Trade and Industry based on Budapest Treaty, as deposition number of FERM BP-2228.

The coryneform bacteria of the present invention can be obtained by enhancing argininosuccinate synthase activity within cells of such coryneform bacteria having the L-arginine-producing ability as mentioned above. The aforementioned argininosuccinate synthase is preferably an argininosuccinate synthase derived from coryneform bacteria. Specifically, the enhancement of the intracellular argininosuccinate synthase activity can be achieved by increasing copy number of a gene coding for the argininosuccinate synthase (*argG*), for example, a gene coding for an argininosuccinate synthase derived from coryneform bacteria (*argG* derived from coryneform bacteria will occasionally be referred to as simply "*argG*" hereinafter), or by modifying an expression regulation sequence so that expression of the gene on a chromosome should be enhanced. The copy number of *argG* in the cells may be increased through transformation of a host of coryneform bacteria by introducing a recombinant DNA which is produced by ligating a DNA fragment containing the *argG* to a vector autonomously replicable in coryneform bacteria, preferably a multi-copy type vector, into the host cell. As a result of the increased copy number of *argG* in the cells of the transformant strain, the argininosuccinate synthase activity is enhanced.

Increasing the copy number of *argG* in a cell can also be achieved by introducing multiple copies of the *argG* gene into the chromosomal DNA of the above-described host strains. In order to introduce multiple copies of the *argG* gene in the chromosomal DNA of bacterium belonging to the genus Corynebacterium, the homologous recombination is carried out using a sequence whose multiple copies exist in the chromosomal DNA as targets. As sequences whose multiple copies exist in the chromosomal DNA, repetitive DNA, inverted repeats exist at the end of a transposable element can be used. Also, as disclosed in Japanese Patent Publication Laid-Open No. 2-109985, it is possible to incorporate the *argG* gene into transposon, and allow it to be transferred to introduce multiple copies of the *argG* gene into the chromosomal DNA. By either method, the number of copies of the *argG* gene within cells of the transformant strain increases, and as a result, argininosuccinate synthase activity is enhanced.

The enhancement of the argininosuccinate synthase activity can also be achieved by, in addition to the aforementioned gene enhancement, modifying an expression regulation sequence so that expression of the gene on a chromosome should be enhanced. Specifically, an expression regulation sequence such as a promoter for *argG* on the chromosomal DNA or a plasmid can be replaced with a stronger one (see Japanese Patent Laid-open No. 1-215280). Strong promoters, which function in cells of coryneform bacteria, include *lac* promoter, *tac* promoter, *trp* promoter, etc. from *Escherichia coli* (Y. Morinaga, M. Tsuchiya, K. Miwa and K. Sano, *J. Biotech*., 5, 305-312 (1987)). In addition, *trp* promoter from a bacterium belonging to the genus *Corynebacterium* is also a preferable promoter (Japanese Patent Laid-open No. 62-195294). Further, a mutant promoter obtained by introducing a mutation enhancing the expression into a promoter peculiar to the *argG* gene can also be used. The replacement with these promoters enhances the expression of *argG*, and thereby the argininosuccinate synthase activity is enhanced. The modification of expression regulation sequence may be combined with the increasing of the copy number of *argG.*

Because the nucleotide sequence of *argG* of coryneform bacteria has been known (nucleotide sequence of ORF: GenBank accession AF030520, nucleotide sequence of a region including ORF and its flanking regions: GenBank accession AF049897), *argG* of coryneform bacteria can be isolated from coryneform bacterial chromosomal DNA by PCR (polymerase chain reaction; see White, T.J. *et al., Trends Genet., 5*,185 (1989)) utilizing primers produced based on the aforementioned nucleotide sequences. Specific examples of such primers include oligonucleotides having the nucleotide sequences shown as SEQ ID NO: 5 and SEQ ID NO: 6. The coryneform bacterium used for the host and the coryneform bacterium that is the source of *argG* may be the same, or may belong to different genera, species or strains.

The vector used for the cloning of *argG* may be a plasmid autonomously replicable in *Escherichia coli* cells, and specific examples thereof include, for example, pUC19, pUC18, pBR322, pHSG299, pHSG298, pHSG399, pHSG398, RSF1010, pSTV29 and the like. A phage vector may also be used. A shuttle vector autonomously replicable in coryneform bacteria and *Escherichia coli* is preferably used for the cloning of *argG* or the introduction of *argG* into coryneform bacteria. As examples of the plasmid autonomously replicable in coryneform bacteria, for example, the following plasmids can be mentioned.
pAM330 (see Japanese Patent Laid-open No. 58-67699)
pHM1519 (see Japanese Patent Laid-open No. 58-77895)
pAJ655 (see Japanese Patent Laid-open No. 58-192900)
pAJ611 (see the same)
pAJ1844 (see the same)
pCG1 (see Japanese Patent Laid-open No. 57-134500)
pCG2 (see Japanese Patent Laid-open No. 58-35197)
pCG4 (see Japanese Patent Laid-open No. 57-183799)
pCG11 (see the same)
pHK4 (see Japanese Patent Laid-open No. 5-7491)

The shuttle vector can be produced by inserting a replication origin derived from a vector autonomously replicable in a coryneform bacterial cell into a vector autonomously replicable in an *Escherichia coli* cell.

In order to prepare recombinant DNA by ligating the gene fragment and a vector, the vector is digested by restriction enzyme(s) corresponding to the termini of the gltBD gene. Ligation is generally performed by using a ligase such as T4 DNA ligase.

The methods to perform, for example, preparation of the genomic DNA, PCR, preparation of plasmid DNA, digestion and ligation of DNA, transformation, design of oligonucleotide used for primers are described by Sambrook, J., Fritsche, E. F., Maniatis, T. in Molecular Cloning, Cold Spring Harbor Laboratory Press (1989).

Transformation may be performed by using, for example, a method in which recipient cells are treated with calcium chloride to increase the permeability of DNA, as reported for *Escherichia coli* K-12 (Mandel, M. and Higa, A., J. Mol. Biol., 53, 159 (1970)), or a method in which competent cells are prepared from cells at the proliferating stage to introduce DNA , as reported for Bacillus subtilis (Duncan, C. H., Wilson, G. A. and Young, F. E., Gene, 1, 153 (1977)).
Alternatively, it is also possible to apply a method in which DNA recipient cells are allowed to be in a state of protoplasts or spheroplasts capable of incorporating recombinant DNA with ease to introduce recombinant DNA into the DNA recipient cells, as known for Bacillus subtilis, actinomycetes, and yeasts (Chang, S. and Choen, S. N., Molec. Gen. Genet., 168, 111 (1979); Bibb, M. J., Ward, J. M. and Hopwood, O. A., Nature, 274, 398 (1978); Hinnen, A., Hicks, J. B. and Fink, G. R., Proc. Natl. Acad. Sci. USA, 75, 1929 (1978)). The electric pulse method (refer to Japanese Patent Publication Laid-Open No. 2-207791) is also applicable.

It is considered that, by enhancing the argininosuccinate synthase activity in microbial cells as described above, the L-arginine biosynthesis can be enhanced, and thereby the amount of L-arginine production can be increased.

Furthermore, the enhancement of the argininosuccinate synthase activity can also be attained by subjecting coryneform bacteria to a mutagenesis treatment and selecting a mutant strain exhibiting high activity of argininosuccinate synthase, or by subjecting a DNA comprising the *argG* gene to a mutagenesis treatment, selecting a mutant gene coding for an argininosuccinate synthase of high activity, and introducing the mutant gene into a coryneform bacterium. As an agent for the mutagenesis treatment of DNA, hydroxylamine and the like may be mentioned. Hydroxylamine is a mutagenic chemical agent inducing mutation by replacement of cytosine with thymine through conversion of cytosine into N4-hydroxycytosine. When coryneform bacteria themselves are subjected to a mutagenesis treatment, mutagenesis can be performed by UV irradiation or with an agent usually used for artificial mutagenesis such as N-methyl-N'-nitrosoguanidine (NTG) and nitrous acid.

In the coryneform bacteria of the present invention, in addition to the argininosuccinate synthase activity, activities of other enzymes included in the L-arginine biosynthesis pathway may also be enhanced.

### <2> Method for producing L-arginine

L-Arginine can be efficiently produced by culturing a coryneform bacterium obtained as described above in a medium so that L-arginine should be produced and accumulated in the medium, and collecting the L-arginine from the medium.

The medium may be a well-known medium conventionally used for the production of amino acid by fermentation utilizing coryneform bacteria. That is, it is a usual medium containing a carbon source, a nitrogen source, inorganic ions, and other organic components, as required.

As the carbon source, it is possible to use sugars such as glucose, sucrose, lactose, galactose, fructose or starch hydrolysate; alcohols such as glycerol or sorbitol; or organic acids such as fumaric acid, citric acid or succinic acid.

As the nitrogen source, it is possible to use inorganic ammonium salts such as ammonium sulfate, ammonium chloride or ammonium phosphate; organic nitrogen such as soybean hydrolysate; ammonia gas; or aqueous ammonia.

It is desirable to allow required substances such as vitamin B1 and L-homoserine or yeast extract to be contained in appropriate amounts as organic trace nutrients. Other than the above, potassium phosphate, magnesium sulfate, iron ion, manganese ion and the like are added in small amounts, if necessary.

Cultivation is preferably carried out under an aerobic condition for 1-7 days. The cultivation temperature is preferably controlled at 24°C to 37°C, and pH is preferably controlled at 5-9 during cultivation. Inorganic or organic, acidic or alkaline substances as well as ammonia gas or the like can be used for pH adjustment. Collection of L-arginine from fermented liquor is usually carried out by combining an ion exchange resin method and other known methods.

### Best Mode for Carrying out the Invention

The present invention will further be explained more specifically with reference to the following examples.

### Example 1

### <1> Cloning of argG gene

In order to amplify the *argG* gene of *Brevibacterium flavum* by PCR, nucleotide sequences of the upstream and downstream regions of the ORF in the gene were determined. The nucleotide sequencing was performed by using primers prepared based on a known nucleotide sequence of *Corynebacterium glutamicum argG* gene ORF (GenBank accession AF030520), and *In vitro* LA PCR cloning kit (produced by TAKARA SHUZO CO., LTD.) in accordance with the attached description of the kit. Specifically, the oligonucleotides having the nucleotide sequences shown in SEQ ID NOS: 1 and 2 (Primers 1 and 2) were used for the region upstream from the ORF, and oligonucleotides having the nucleotide sequences shown in SEQ ID NOS: 3 and 4 (Primers 3 and 4) were used for the region downstream from the ORF, respectively. The chromosomal DNA of the 2247 strain (ATCC14067), which is a wild strain of *Brevibacterium flavum, was* fully digested with a restriction enzyme *Eco*RI*,* and a primary PCR was performed with Primer 2 or 3, and a secondary PCR with Primer 1 or 4 to determine the nucleotide sequences of the upstream and downstream regions of *argG*.

Based on the nucleotide sequences determined as described above, oligonucleotides having the nucleotide sequences shown in SEQ ID NO: 5 and 6 (Primers 5 and 6) were synthesized, and PCR was performed by using chromosomal DNA of *Brevibacterium flavum* 2247 strain as template. The PCR reaction was performed for 25 cycles each consisting of reactions at 94°C for 30 seconds, 55°C for 1 second, and 72°C for 2 minutes and 30 seconds. The obtained DNA fragment was cloned into the *Sma*I site in the multi-cloning site of the cloning vector pSTV29 (produced by TAKARA SHUZO CO., LTD.) to produce pSTVargG.

Furthermore, a replication origin of the plasmid pHM1519 autonomously replicable in Coryneform bacteria (Japanese Patent Laid-open No. 5-7491), which plasmid had been already obtained (Miwa, K. *et al.*, *Agric. Biol. Chem., 48,* 2901-2903 (1984)), was introduced into the pSTVargG. Specifically, pHM1519 was digested with restriction enzymes *Bam*HI and *Kpn*I to obtain a gene fragment containing the replication origin, and the obtained fragment was blunt-ended by using Blunting kit produced by TAKARA SHUZO CO., LTD., and inserted into the *Sal*I site of the pSTVargG using *Sal*I linker (produced by TAKARA SHUZO CO., LTD. make) to produce pargG.

### <2> Introduction of pargG into coryneform bacteria

The plasmid pargG was introduced into *Brevibacteriu*m *flavum* AJ11169 strain, AJ11336 strain and AJ11345 strain, and *Corynebacterium glutamicum* AJ12092 strain and AJ12430 strain.

The introduction of the plasmid was performed by using the electric pulse method (Japanese Patent Laid-open No. 2-207791). The transformant of each strain into which pargG was introduced was selected as a chloramphenicol resistant strain on a CM2G plate medium (containing 10 g of polypeptone, 10 g of yeast extract, 5 g of glucose, 5 g of NaCl, and 15 g of agar in 1 L of pure water, pH 7.2) containing 4 µg/ml of chloramphenicol.

### <3> Production of L-arginine by pargG-introduced strain

The aforementioned transformants, and AJ11169 strain, AJ12092 strain, AJ11336 strain, AJ11345 strain, and AJ12430 strain were each plated on an agar medium containing 0.5 g/dl of glucose, 1 g/dl of polypeptone, 1 g/dl of yeast extract, 0.5 g/dl of NaCl, and 5 µg/l of chloramphenicol, and cultured at 31.5°C for 20 hours. One platinum loop of the resulting bacterial cells were inoculated into a medium containing 4 g/dl of glucose, 6.5 g/dl of ammonium sulfate, 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), and cultured at 31-5°C for 50 hours in a flask with shaking. The culture was performed for less than 120 hours for the 11169 strain, or for less than 48 hours for the other strains. Each culture broth was diluted 51 times with 0.2 N HCl, and absorbance at 620 nm (CD₆₂₀) of the diluted medium and the amount of produced L-arginine (g/dl) were measured. The results are shown in Table 1.

As a result of the measurement, it was confirmed that citrulline and ornithine were simultaneously produced in the media for the AJ11169 strain, AJ12092 strain, AJ11336 strain, AJ11345 strain, and AJ12430 strain. On the other hand, citrulline and ornithine were reduced and L-arginine yield was improved in the media for those strains transformed with pargG. Glucose in the medium was substantially completely consumed for all of the strain.

**Table 1**

| Strain | OD₆₂₀ | Arginine (g/dl) | Ctrulline (mg/dl) | Ornithine (mg/dl) |
|---|---|---|---|---|
| 11169 | 0.702 | 0.38 | 8.1 | 3.3 |
| 11169/pargG | 0.710 | 0.39 | 0.8 | 2.5 |
| 12092 | 0.502 | 1.25 | 171.7 | 37.0 |
| 12092/pargG | 0.520 | 1.47 | 6.4 | 2.1 |
| 11336 | 0.531 | 0.71 | 122.6 | 22.6 |
| 11336/pargG | 0.540 | 0.86 | 8.7 | 2.3 |
| 11345 | 0.667 | 1.33 | 97.6 | 15.1 |
| 11345/pargG | 0.670 | 1.45 | 3.3 | 3.3 |
| 12430 | 0.969 | 0.52 | 5.4 | 0.1 |
| 12430/pargG | 0.945 | 0.53 | 0.7 | 0.0 |

### Example 2

### <1> Nucleotide sequence determination of upstream region of argG gene

The upstream and downstream regions from ORF were cloned based on the nucleotide sequence of the ORF region of the *argG* gene (GenBank AF030520). The upstream and downstream regions were cloned by using Primers 1, 2, 3, and 4, and *In vitro* LA PCR cloning kit (produced by TAKARA SHUZO CO., LTD.).

The chromosomal DNA of *Brevibacterium flavum* 2247 strain was fully digested with a restriction enzyme *Eco*RI, and a primary PCR was performed with Primer 2 or 3, and a secondary PCR with Primer 1 or 4 by using the aforementioned kit to determine the nucleotide sequences of the upstream and downstream regions of the *argG.*

### <2> Estimation of promoter site

From the aforementioned sequences, a promoter-like sequence in the upstream of ORF of the *argG* gene was searched by using commercially available software (GENETYX). The mutation was introduced into the site exhibiting the highest homology score (about 120 bp upstream from the first ATG), and promoter activity was determined.

### <3> Introduction of mutation into promoter sequence and activity measurement for mutant promoters

Various mutations were introduced into the site exhibiting the highest score in the upstream of the ORF of *argG* gene by using primers having the nucleotide sequences shown in SEQ ID NOS: 9-13 (Primer 9, Primer 10, Primer 11, Primer 12 or Primer 13) and SEQ ID NO: 7 (Primer 7) for introducing mutation. The 1st PCR was performed by using the chromosomal DNA of *Corynebacterium glutamicum* AJ12092 strain as template. By using the product of this PCR as the 3' end primer, and primer having the nucleotide sequence shwon in SEQ ID NO: 8 (Primer 8) as the 5' primer, PCR was performed again with the same chromosomal DNA as template to obtain DNA fragments with various mutations introduced into the target promoter region.

Then, in order to measure activity of the aforementioned mutant promoters, each of those DNA fragments was inserted into the *Sma*I site of a promoter probe vector pNEOL so that it should be in the correct order with a reporter gene, *lacZ*, to obtain plasmids pNEOL-1, pNEOL-2, pNEOL-3, pNEOL-4, and pNEOL-7. Further, as a control for the promoter activity, a DNA fragment obtained by performing PCR using Primers 7 and 8, and the chromosomal DNA of the AJ12092 strain as template was similarly inserted into the upstream region from the *lacZ* gene of pNEOL to construct a plasmid pNEOL-0.

pNEOL-0, pNEOL-1, pNEOL-2, pNEOL-3, pNEOL-4, and pNEOL-7 were each introduced into the *Corynebacterium glutamicum* AJ12092 strain. The introduction of the plasmids was performed by using the electric pulse method (Japanese Patent Laid-open No. 2-207791). The transformants were selected as chloramphenicol resistant strains on a CM2G plate medium (containing 10 g of polypeptone, 10 g of yeast extract, 5 g of glucose, 5 g of NaCl, and 15 g of agar in 1 L of pure water, pH 7.2) containing 4 µg/ml of chloramphenicol.

These strains were each plated on an agar medium containing 0.5 g/dl of glucose, 1 g/dl of polypeptone, 1 g/dl of yeast extract, 0.5 g/dl of NaCl, and 5 µg/l of chloramphenicol, and cultured at 31.5°C for 20 hours. One platinum loop of the resulting bacterial cells were inoculated into a medium containing 3 g/dl of glucose, 1.5 g/dl of ammonium sulfate, 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), and cultured at 31.5°C for 18 hours. β-Galactosidase activity was measured for the obtained cells.

Since the β-galactosidase activity was detected for AJ12092/pNEOL-0 as shown in Table 2, it was found that the DNA fragment inserted in the upstream of the *lacZ* structural gene functioned as a promoter. Further, as compared with AJ12092/pNEOL-0, the other strains introduced with the plasmids showed higher β-galactosidase activity, and it was revealed that introduction of mutation into this promoter-like sequence promoted the transcription activity as shown in Table 2. Among these, the mutations of pNEOL-3 and pNEOL-7 were decided to be introduced into the promoter-like sequence in the *argG* gene upstream region on the chromosome of L-arginine-producing bacterium AJ12092 strain by homologous recombination.

**Table 2**

| Relative activity (AJ12092/pNEOL-0 = 1) | |
|---|---|
| AJ12092 | nd |
| AJ12092/pNEOL-0 | 1.0 |
| AJ12092/pNEOL-1 | 2.8 |
| AJ12092/pNEOL-2 | 2.7 |
| AJ12092/pNEOL-3 | 1.8 |
| AJ12092/pNEOL-4 | 0.8 |
| AJ12092/pNEOL-7 | 3.0 |

### <4> Construction of plasmid for introducing mutation

A DNA fragment which contained an upstream region containing 5' sequence of the coding region of the *argG* gene and the promoter of the *argG* gene, and the 3' region of the *argR* gene, which was obtained by performing PCR with primers having the nucleotide sequence of SEQ ID Nos: 14 and 15 (Primers 14 and 15) and the chromosomal DNA of the AJ12092 strain as template, was inserted into the multi-cloning site of the cloning vector pHSG398 (produced by TAKARA SHUZO CO., LTD.) to construct a plasmid p0. Then, a DNA fragment obtained by digesting p0 with restriction enzymes *Eco*RV and *Bsp*HI, and a DNA fragment obtained by digesting pNEOL-3 and pNEOL-7 with restriction enzymes *Eco*RV and *Bsp*HI were ligated to obtain plasmids for introducing mutation, p3 (mutation derived from Primer 11 for introducing mutation) and p7 (mutation derived from Primer 13 for introducing mutation).

The geometry of the *argG* gene and its flanking sequences with respect to each primer is shown in Fig. 1.

### <5> Introduction of plasmid for introducing mutation into L-arginine-producing bacterium

Each of the aforementioned plasmids was introduced into L-arginine-producing bacterium, *Corynebacterium glutamicum* AJ12092 strain. The introduction of the plasmids was performed by using the electric pulse method (Japanese Patent Laid-open No. 2-207791). Since these plasmids could not autonomously replicate in *Brevibacterium flavum,* only the strains in which each plasmid was incorporated into the chromosome by homologous recombination could be selected as a chloramphenicol resistant strain. The strains into which a plasmid for introducing mutation was introduced were selected as chloramphenicol resistant strains on a CM2G plate medium (containing 10 g of polypeptone, 10 g of yeast extract, 5 g of glucose, 5 g of NaCl, and 15 g of agar in 1 L of pure water, pH 7.2) containing 5 µg/ml of chloramphenicol- Then, among strains made chloramphenicol sensitive through another homologous recombination, strains were selected in which the promoter region of the *argG* gene was replaced with a desired mutant sequence.

As a result, there were obtained a strain in which the *argG* gene promoter on the chromosome was replaced with the sequence of p3 (AJ12092-P3), and a strain in which the *argG* gene promoter on the chromosome was replaced with the sequence of p7 (AJ12092-P7).

### <6> Argininosuccinate synthase (ArgG) activity of promoter-mutant strains

The ArgG activity was measured for the aforementioned two kinds of the *argG* promoter mutants (AJ12092-P3 and AJ12092-P7) and the *Corynebacterium glutamicum* (AJ12092/pargG) in which the *argG* gene obtained in Example 1 was amplified by the plasmid. These strains were each plated on an agar medium containing 0.5 g/dl of glucose, 1 g/dl of polypeptone, 1 g/dl of yeast extract, 0.5 g/dl of NaCl, and 5 µg/l of chloramphenicol, and cultured at 31.5°C for 20 hours. One platinum loop of the resulting bacterial cells were inoculated into a medium containing 3 g/dl of glucose, 1.5 g/dl of ammonium sulfate, 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), and cultured at 31.5°C for 18 hours. For the resulting bacterial cells, the ArgG activity was measured according to the previously reported method (*Journal of General Microbiology* (1990), *136,* 1177-1183).

The ArgG activity of the aforementioned two kinds of *argG* promoter mutants and *argG*-amplified strain (AJ12092/pargG) is shown in Table 3. As shown in Table 3, by introducing a mutation into the promoter, the ArgG activity was increased about twice in AJ12092-P3, and about 3 times in AJ12092-P7 compared with the parent strain. Further, the ArgG activity in the AJ12092/pargG was about 4.5 times higher than the parent strain.

**Table 3**

| | Relative activity (AJ12092 = 1) |
|---|---|
| AJ12092 | 1.0 |
| AJ12092-P3 | 2.1 |
| AJ12092-P7 | 2.9 |
| AJ12092/pargG | 4.4 |

### <7> L-arginine production by promoter-mutant strains

The *argG* promoter-mutant strains were cultured in flasks. As control, the parent strain AJ12092 and the *argG* amplified strain AJ12092/pargG were cultured in the same manner. Each of the strains was inoculated into a medium containing 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), plated on an agar medium containing 0.5 g/dl of glucose, 1 g/dl of polypeptone, 1 g/dl of yeast extract, 0.5 g/dl of NaCl, and 5 µg/l of chloramphenicol, and cultured at 31.5°C for 20 hours. One platinum loop of the resulting bacterial cells were inoculated into 20 ml of a medium containing 4 g/dl of glucose, 6.5 g/dl of ammonium sulfate, 0.1 g/dl of KH₂PO₄, 0.04 g/dl of MgSO₄, 0.001 g/dl of FeSO₄, 0.01 g/dl of MnSO₄, 5 µg/dl of vitamin B₁, 5 µg/dl of biotin and soybean hydrolysate (45 mg/dl in terms of the amount of N), and cultured in flasks at 31.5°C until the glucose was completely consumed. Each culture broth was diluted 51 times with 0.2 N HCl, and absorbance at 620 nm (OD₆₂₀) of the diluted medium and the amount of produced L-arginine were measured (concentration, g/dl). The results are shown in Table 4.

**Table 4**

| | OD₆₂₀ | Arginine (g/dl) |
|---|---|---|
| AJ12092 | 0.502 | 1.25 |
| AJ12092-P3 | 0.510 | 1.47 |
| AJ12092-P7 | 0.514 | 1.43 |

## Claims

1. A coryneform bacterium having L-arginine-producing ability in which an activity of intracellular argininosuccinate synthase is enhanced.

2. The bacterium of claim 1, wherein the argininosuccinate synthase is derived from a coryneform bacterium.

3. The bacterium of claim 1 or 2, wherein the activity of intracellular argininosuccinate synthase is enhanced by increasing copy number of a gene which codes for an argininosuccinate synthase derived from a coryneform bacterium in the bacterial cell, or modifying an expression regulation sequence for the gene in the bacterial cell so that expression of the gene should be enhanced.

4. A method for producing L-arginine, comprising the steps of culturing the bacterium of any one of claims 1-3 in a medium to produce and accumulate L-arginine, and collecting the L-arginine from the medium.
